# EUROPEAN PATENT SPECIFICATION

(11) **EP 4 077 709 B1**
(45) Date of publication and mention of the grant of the patent: **18.06.2025**
(21) Application number: 20902209.4
(22) Date of filing: 17.12.2020
(51) Int. Cl.: C12Q 1/68, G01N 33/574, G01N 1/18, G01N 33/566, A61B 46/23, G01N 33/53, A61B 17/20, A61K 33/24, A61K 33/00, A61K 49/04

(54) **METHOD OF MEASURING CELL-FREE DNA IN SURGICAL DRAIN FLUID TO SELECT ADJUVANT THERAPY**
VERFAHREN ZUM MESSEN DER ZELLFREIEN DNA IN EINEM CHIRURGISCHEN ABFLUSSFLUID ZUR AUSWAHL DER ADJUVANSTHERAPIE
PROCÉDÉ DE MESURE D'ADN ACELLULAIRE DANS UN FLUIDE DE DRAINAGE CHIRURGICAL POUR SÉLECTIONNER UNE THÉRAPIE ADJUVANTE

(30) Priority: 16.12.2019 US 201962948686 P
(43) Date of publication of application: 26.10.2022
(73) Proprietor: Washington University, St. Louis, Missouri 63130 (US)
(72) Inventor: ZEVALLOS, Jose, St. Louis, Missouri 63130 (US); RAMIREZ, Ricardo, St. Louis, Missouri 63130 (US)
(74) Representative: Beck Greener LLP
(86) International application number: PCT/US2020/065446
(87) International publication number: WO 2021/127065

(56) References cited:
- WO-A1-2019/055835
- WO-A1-2019/133391
- US-A1- 2013 137 593
- US-A1- 2015 315 289
- BROGGI MARIA A.S. ET AL: "Tumor-associated factors are enriched in lymphatic exudate compared to plasma in metastatic melanoma patients", JOURNAL OF EXPERIMENTAL MEDICINE, vol. 216, no. 5, 6 May 2019 (2019-05-06), US, pages 1091 - 1107, XP093113131, ISSN: 0022-1007, Retrieved from the Internet <URL:https://rupress.org/jem/article-pdf/216/5/1091/1761715/jem_20181618.pdf> DOI: 10.1084/jem.20181618
- TSUJINAKA SHINGO, KONISHI FUMIO: "Drain vs No Drain After Colorectal Surgery", INDIAN JOURNAL OF SURGICAL ONCOLOGY, SPRINGER INDIA, INDIA, vol. 2, no. 1, 1 March 2011 (2011-03-01), India, pages 3 - 8, XP055843064, ISSN: 0975-7651, DOI: 10.1007/s13193-011-0041-2
- MASAGO KATSUHIRO, FUJITA SHIRO, YATABE YASUSHI: "Targeting minimal residual disease after surgery with molecular targeted therapy: the real path to a cure?", JOURNAL OF THORACIC DISEASE, CHINA, vol. 10, no. S16, 1 June 2018 (2018-06-01), China, pages S1982 - S1985, XP055843067, ISSN: 2072-1439, DOI: 10.21037/jtd.2018.04.155
- LASSIG AMY ANNE D., JOSEPH ANNE M., LINDGREN BRUCE R., YUEH BEVAN: "Association of Oral Cavity and Oropharyngeal Cancer Biomarkers in Surgical Drain Fluid With Patient Outcomes", JAMA OTOLARYNGOLOGY–HEAD & NECK SURGERY, AMERICAN MEDICAL ASSOCIATION, US, vol. 143, no. 7, 1 July 2017 (2017-07-01), US, pages 670, XP055843081, ISSN: 2168-6181, DOI: 10.1001/jamaoto.2016.3595

## Description

### CROSS-REFERENCE TO RELATED APPLICATIONS

This application claims priority from U.S. Provisional Application Serial No. 62/948,686 filed on December 16, 2019.

### STATEMENT REGARDING FEDERALLY SPONSORED RESEARCH OR DEVELOPMENT

Not applicable.

### FIELD OF THE DISCLOSURE

The present disclosure generally relates to methods of diagnosing and treating high-risk cancers of the neck and throat.

### BACKGROUND OF THE DISCLOSURE

For patients undergoing surgical treatments of various cancers, there are currently no effective methods of determine if any cancerous tissues were left behind after surgery. As a consequence, physicians must define post-surgery treatments such as radiation or chemo based on limited information, affecting patient outcomes and quality of life.

Over the last five years, there has been growing interest in the development of liquid biopsies for cancer diagnosis and treatment.

Recently, several groups have sought to develop a cell-free DNA (cfDNA) liquid biopsy test for early detection of recurrence in HPV(+) head and neck cancer by detecting HPV DNA associated with circulating tumor cells (ctHPVDNA) in plasma. Many of these tests have relied on measurement of tumor-derived cell-free DNA (cfDNA) in plasma as a predictor of patients susceptible to cancer or as a marker of tumor recurrence. Although ctHPVDNA testing in plasma is promising as an early marker of recurrence, it may not yet be a reliable prognostic tool but could also be used potentially to define the extent of treatment necessary as a way of de-intensifying therapy.

However, the detection of plasma analytes, such as ctDNA or cfDNA, is ill-suited for post-surgery monitoring for residual cancer at surgical sites given the characteristics of the circulatory system. Given the systemic nature of circulation, the detection of cfDNA in plasma is relatively non-specific and does not provide the ability to determine whether the source of the ctDNA or cfDNA is the surgical site. Further, the dilution of analytes within a patient's plasma volume results in relatively low concentrations of analytes in plasma samples, increasing the challenge of detection. In addition, the relatively long latency of the appearance of cfDNA in the plasma results in a lag between the release of ctDNA or cfDNA from the surgical site. As a consequence, post-surgical treatments may be delayed, potentially affecting patient outcomes and quality of life. WO2019/055835 discloses a method for detecting somatic single nucleotide variants (SNVs) from cell-free nucleic acid, such as deoxyribonucleic acid (cfDNA) and ribonucleic acid (cfRNA) for detection of minimal residual disease.

### SUMMARY OF THE DISCLOSURE

In one aspect, a method for detecting minimal residual disease in a subject following a cancer surgery is disclosed. The method includes obtaining a sample from the subject. The sample includes a surgical drainage. The method also includes isolating an amount of tumor-associated genetic material from the sample, sequencing the amount of tumor-associated genetic material to detect and quantify at least one tumor-associated mutation or variant in the amount of tumor-associated genetic material, and providing the at least one quantity of the at least one tumor-associated mutation or variant to a practitioner. The at least one tumor-associated mutation or variant is indicative of minimal residual disease in the subject. In some aspects, the amount of tumor-associated genetic material includes cell-free DNA, RNA, proteins, exosomes, and any combination thereof. In some aspects, isolating the amount of tumor-associated genetic material from the sample further includes filtering the sample, centrifuging the sample, contacting the sample with a chromatography medium, and any combination thereof. In some aspects, the method further includes selecting an additional treatment based on the quantity of the at least one tumor-associated mutation or variant. In some aspects, the additional treatment is selected from radiotherapy, chemotherapy, follow-up surgery, active surveillance with imaging, and any combination thereof. In some aspects, the tumor-associated genetic material is produced by a plurality of cancer cells. In some aspects, the plurality of cancer cells are selected from one of oropharyngeal cancer cells, lung cancer cells, breast cancer cells, melanoma cells, colon cancer cells, thyroid cancer cells, prostate cancer cells, ovarian cancer cells, testicular cancer cells, penile cancer cells, cervical cancer cells, anal cancer cells, brain cancer cells, liver cancer cells, pancreatic cancer cells, and testicular cancer cells. In some aspects, the cancer surgery is selected from a resectioning surgery, a dissection surgery, an excision surgery, and any combination thereof. In some aspects, obtaining the sample from the subject further includes capturing a surgical drainage from a drainage tube associated with the cancer surgery. In some aspects, obtaining the sample from the subject further includes capturing a surgical drainage from the drainage tube within about 24 hours of the cancer surgery. In some aspects, the tumor-associated genetic material includes cell-free HPV DNA (cfDNA) associated with oropharyngeal cancer cells. In some aspects, sequencing the amount of tumor-associated genetic material to detect and quantify at least one tumor-associated mutation or variant further includes subjecting the sample to a sequencing method selected from next generation DNA sequencing, next generation RNA sequencing, next generation protein sequencing, PCR, Western blot, and any combination thereof. The method of any preceding claim, wherein sequencing the amount of tumor-associated genetic material to detect and quantify at least one tumor-associated mutation or variant in the amount of tumor-associated genetic material further comprises detecting and quantifying at least one HPV strain associated with HPV(+) oropharyngeal cancer comprising HPV16 DNA, HPV18 DNA, HPV31 DNA, HPV33 fragment, HPV35 fragment, HPV45 DNA, HPV52 DNA, HPV58 DNA, and any combination thereof.

In another aspect, a method for selecting a post-operative treatment for a cancer patient in need is disclosed. The method includes obtaining a sample from the subject. The sample includes a surgical drainage. The method also includes isolating an amount of tumor-associated genetic material from the sample, sequencing the amount of tumor-associated genetic material to detect and quantify at least one tumor-associated mutation or variant in the amount of tumor-associated genetic material, providing the at least one quantity of the at least one tumor-associated mutation or variant to a practitioner, and selecting an additional treatment based on the quantity of the at least one tumor-associated mutation or variant. The at least one tumor-associated mutation or variant is indicative of minimal residual disease in the subject. In some aspects, the amount of tumor-associated genetic material includes cell-free DNA, RNA, proteins, exosomes, and any combination thereof. In some aspects, isolating the amount of tumor-associated genetic material from the sample further includes filtering the sample, centrifuging the sample, contacting the sample with a chromatography medium, and any combination thereof. In some aspects, the additional treatment is selected from radiotherapy, chemotherapy, follow-up surgery, active surveillance with imaging, and any combination thereof. In some aspects, the tumor-associated genetic material is produced by a plurality of cancer cells. In some aspects, the plurality of cancer cells are selected from one of oropharyngeal cancer cells, lung cancer cells, breast cancer cells, melanoma cells, colon cancer cells, thyroid cancer cells, prostate cancer cells, ovarian cancer cells, testicular cancer cells, penile cancer cells, cervical cancer cells, anal cancer cells, brain cancer cells, liver cancer cells, pancreatic cancer cells, and testicular cancer cells. In some aspects, the cancer surgery is selected from a resectioning surgery, a dissection surgery, an excision surgery, and any combination thereof. In some aspects, obtaining the sample from the subject further includes capturing a surgical drainage from a drainage tube associated with the cancer surgery. In some aspects, obtaining the sample from the subject further includes capturing a surgical drainage from the drainage tube within about 24 hours of the cancer surgery. In some aspects, the tumor-associated genetic material includes cell-free HPV DNA (cfDNA) associated with oropharyngeal cancer cells. In some aspects, sequencing the amount of tumor-associated genetic material to detect and quantify at least one tumor-associated mutation or variant further includes subjecting the sample to a sequencing method selected from next generation DNA sequencing, next generation RNA sequencing, next generation protein sequencing, PCR, Western blot, and any combination thereof. The method of any preceding claim, wherein sequencing the amount of tumor-associated genetic material to detect and quantify at least one tumor-associated mutation or variant in the amount of tumor-associated genetic material further comprises detecting and quantifying at least one HPV strain associated with HPV(+) oropharyngeal cancer comprising HPV16 DNA, HPV18 DNA, HPV31 DNA, HPV33 fragment, HPV35 fragment, HPV45 DNA, HPV52 DNA, HPV58 DNA, and any combination thereof.

Other objects and features will be in part apparent and in part pointed out hereinafter.

### DESCRIPTION OF THE DRAWINGS

Those of skill in the art will understand that the drawings, described below, are for illustrative purposes only. The drawings are not intended to limit the scope of the present teachings in any way.
FIG. 1 is a flow chart illustrating a method of detecting minimal residual disease after a surgery in accordance with one aspect of the disclosure.
FIG. 2 is a schematic illustration of a method of isolating exosomes from a surgery drainage sample in accordance with one aspect of the disclosure.
FIG. 3A is an image of exosomes isolated from a surgery drain fluid using ulracentrifugation.
FIG. 3B is an image of exosomes isolated from a surgery drain fluid using ulracentrifugation using the method illustrated in FIG. 2.

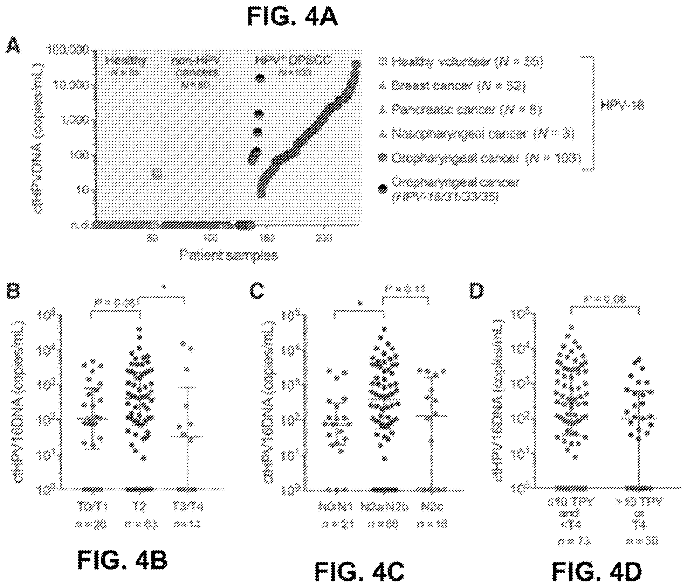

FIG. 4A is a graph comparing levels of HPV DNA associated with circulating tumor cells (ctHPVDNA) detected in the plasma of healthy patients, non-HP-associated cancer patients, and HPV(-)/HPV(+) oropharyngeal cancer patients.
FIG. 4B is a graph comparing levels of HPV16 DNA associated with circulating tumor cells (ctHPV16DNA) detected in the plasma of oropharyngeal cancer patients at different tumor stages.
FIG. 4C is a graph comparing levels of HPV16 DNA associated with circulating tumor cells (ctHPV16DNA) detected in the plasma of oropharyngeal cancer patients with different tumor stages and/or tobacco pack year (TPY) scores.
FIG. 5A is an image of an exosome prep obtained from surgery drain fluid of a patient with HPV positive oropharyngeal cancer.
FIG. 5B is an image of an exosome prep obtained from surgery drain fluid of a patient with HPV positive oropharyngeal cancer.
FIG. 5C is an image of an exosome prep obtained from surgery drain fluid of a patient with HPV positive oropharyngeal cancer.
FIG. 5D is an image of an exosome prep obtained from surgery drain fluid of a patient with HPV positive oropharyngeal cancer.
FIG. 5E is an image of an exosome prep obtained from surgery drain fluid of a patient with HPV positive oropharyngeal cancer.
FIG. 5F is an image of an exosome prep obtained from surgery drain fluid of a patient with HPV positive oropharyngeal cancer.
FIG. 5G is an image of an exosome prep obtained from surgery drain fluid of a patient with HPV positive oropharyngeal cancer.
FIG. 5H is an image of an exosome prep obtained from surgery drain fluid of a patient with HPV positive oropharyngeal cancer.
FIG. 5I is an image of an exosome prep obtained from surgery drain fluid of a patient with HPV positive oropharyngeal cancer.
FIG. 5J is an image of an exosome prep obtained from surgery drain fluid of a patient with HPV positive oropharyngeal cancer.
FIG. 6A is a graph comparing the proportion of favorable and unfavorable ctHPV16DNA plasma clearance profiles in oropharyngeal cancer patient profiles identified as low-risk and high-risk.
FIG. 6B is a graph summarizing disease progression in oropharyngeal cancer patients from low/high risk populations with favorable and unfavorable ctHPV16DNA clearance profiles.
FIG. 6C is a survival curve comparing regional disease-free survival over 24 months for oropharyngeal cancer patients from low/high risk populations with favorable and unfavorable ctHPV16DNA clearance profiles.
FIG. 7A is a graph comparing survival curves for oropharyngeal cancer patient populations characterized as negative or positive for extranodal extensions (ENE).
FIG. 7B is a graph comparing survival curves for oropharyngeal cancer patient populations characterized as nodal stages N1 and N2, as well as negative or positive for extranodal extensions (ENE).
FIG. 8A is a graph comparing levels of cell-free HPV DNA obtained from drain fluid following neck dissection surgery for oropharyngeal cancer patient populations characterized as nodal stages N0-N1, and nodal stages N2; differences were observed with a trend toward higher HPV DNA levels with more aggressive disease; no statistical tests were performed due to small sample size.
FIG. 8B is a graph comparing levels of cell-free HPV DNA obtained from drain fluid following neck dissection surgery for oropharyngeal cancer patient populations characterized by extranodal extension (ENE) status; differences were observed with a trend toward higher HPV DNA levels with more aggressive disease; no statistical tests were performed due to small sample size.
FIG. 9 is a graph comparing expression levels of cell-free HPV DNA obtained from drain fluid following neck dissection surgery for oropharyngeal cancer patient populations as a function of the number of HPV+ lymph nodes; HPV DNA level in surgical drain fluid was strongly correlated with the number of positive lymph nodes; Spearman's rho was significant for a strong positive correlation between pathologically confirmed positive lymph nodes and amount of HPV DNA in drain fluid following neck dissection.
FIG. 10 is a graph comparing expression levels of cell-free HPV DNA obtained from ipsilateral and contralateral drain fluid following bilateral neck dissection surgery; lower levels of HPV DNA were detected in the contralateral neck drain with respect to the primary tumor in patients undergoing bilateral neck dissection; number of positive lymph nodes reported on final pathology are displayed above the measured values for the ipsilateral (red circle) and contralateral (blue square) drain fluid samples; HPV DNA was not detectable in any of the samples from necks without nodal disease.
FIGS. 11A is an image of an exosome prep obtained from surgery drain fluid of a patient with HPV negative oropharyngeal cancer.
FIG. 11B is an image of an exosome preps obtained from surgery drain fluid of a patient with HPV negative oropharyngeal cancer.
FIG. 11C is an image of an exosome preps obtained from surgery drain fluid of a patient with HPV negative oropharyngeal cancer.
FIG. 11D is an image of an exosome preps obtained from surgery drain fluid of a patient with HPV negative oropharyngeal cancer.
FIG. 11E is an image of an exosome preps obtained from surgery drain fluid of a patient with HPV negative oropharyngeal cancer.
FIG. 11F is an image of an exosome preps obtained from surgery drain fluid of a patient with HPV negative oropharyngeal cancer.
FIG. 11G is an image of an exosome preps obtained from surgery drain fluid of a patient with HPV negative oropharyngeal cancer.
FIG. 11H is an image of an exosome preps obtained from surgery drain fluid of a patient with HPV negative oropharyngeal cancer.
FIG. 11I is an image of an exosome preps obtained from surgery drain fluid of a patient with HPV negative oropharyngeal cancer.
FIG. 11J is an image of an exosome preps obtained from surgery drain fluid of a patient with HPV negative oropharyngeal cancer.

There are shown in the drawings arrangements, which are presently discussed, it being understood, however, that the present embodiments are not limited to the precise arrangements and are instrumentalities shown. While multiple embodiments are disclosed, still other embodiments of the present disclosure will become apparent to those skilled in the art from the following detailed description, which shows and describes illustrative aspects of the disclosure. As will be realized, the invention is capable of modifications in various aspects, all without departing from the spirit and scope of the present disclosure. Accordingly, the drawings and detailed description are to be regarded as illustrative in nature and not restrictive.

### DETAILED DESCRIPTION OF THE INVENTION

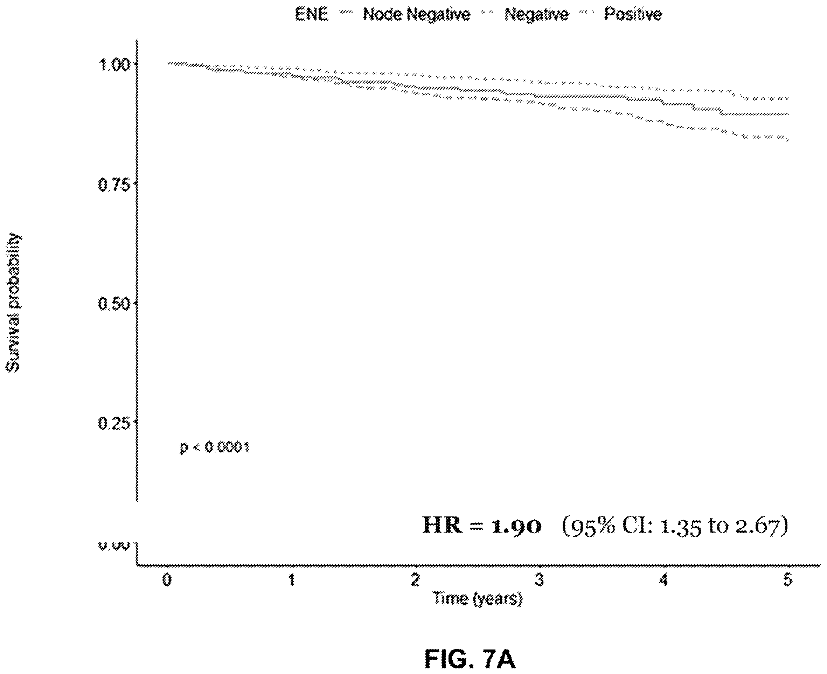

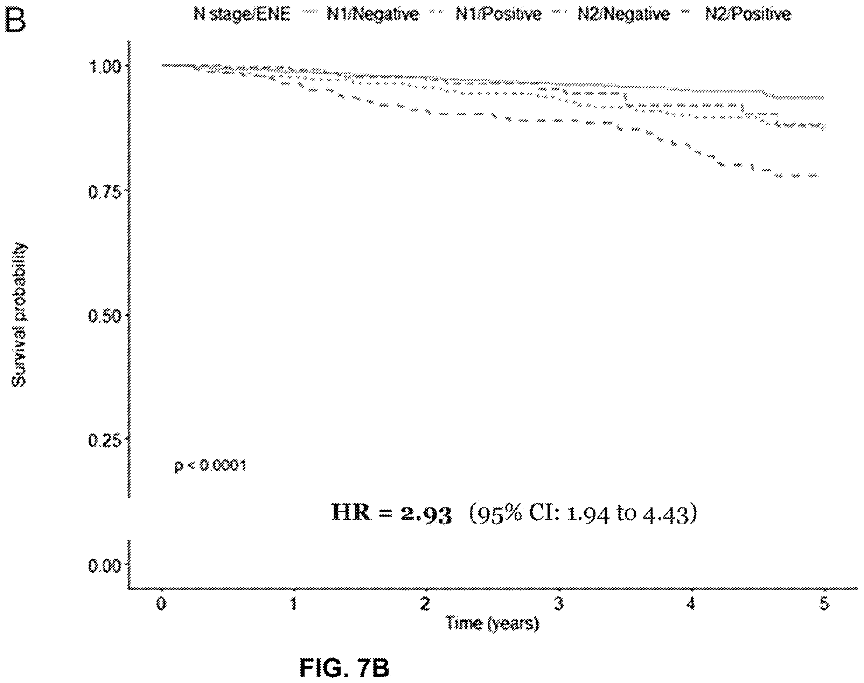

As the incidence of HPV(+) head and neck cancer continues to rise, there is a need to provide objective measures of tumor burden in order to safely provide de-intensified therapy. Currently, assessments of extranodal extensions (ENE) and numbers of positive lymph nodes (node staging) represent the gold standard of prognostic measures obtained after surgical neck dissection, as illustrated in FIG. 7A and FIG. 7B. Both assessments require histological analysis of invasively excised tissues.

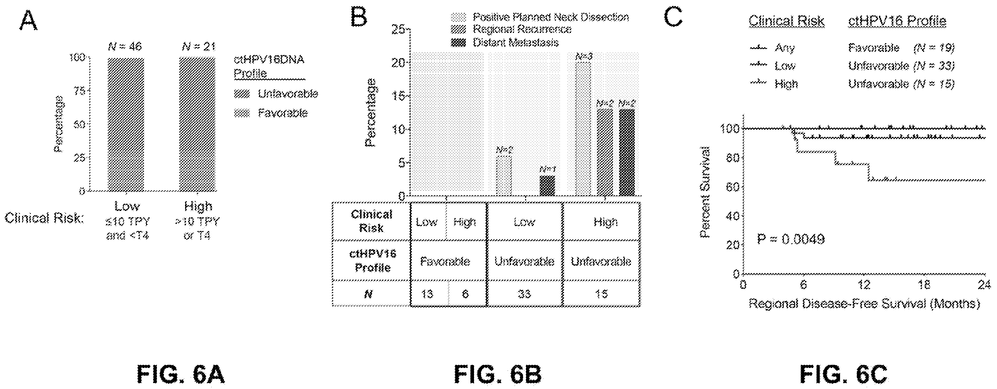

When compared to HPV-negative head and neck cancers, HPV-positive cancers are associated with a much better prognosis (see FIGS. 6A, 6B, and 6C). Without being limited to any particular theory, patients with low HPV16DNA levels are thought to have a higher likelihood of HPV integration, which is associated with poor outcome in HPV(+) cancers. HPV(+) cancers are treated either with transoral surgery or with definitive radiation therapy, and several national trials are focused on de-intensification of treatment in order to reduce side effects. However, to date no objective measures to define inclusion in treatment de-intensification are currently available.

Cell-free DNA and circulating tumor cells (CTCs) obtained from plasma samples have emerged as powerful analytes for identifying resistance to therapy across a variety of cancer types. Preliminary results indicate that plasma-detected HPV DNA may serve as a promising biomarker of recurrence of oropharyngeal cancer, but the reliability of this method remains to be confirmed.

In various aspects, drain fluid samples collected after a surgical neck dissection treatment serve as a more proximal indicator of the post-surgical condition of oropharyngeal tissues, in particular with respect to HPV expression, relative to plasma samples. In various other aspects, HPV DNA in drain fluid could serve as an adjunct objective measure to traditional histopathology and plasma-based cfDNA.

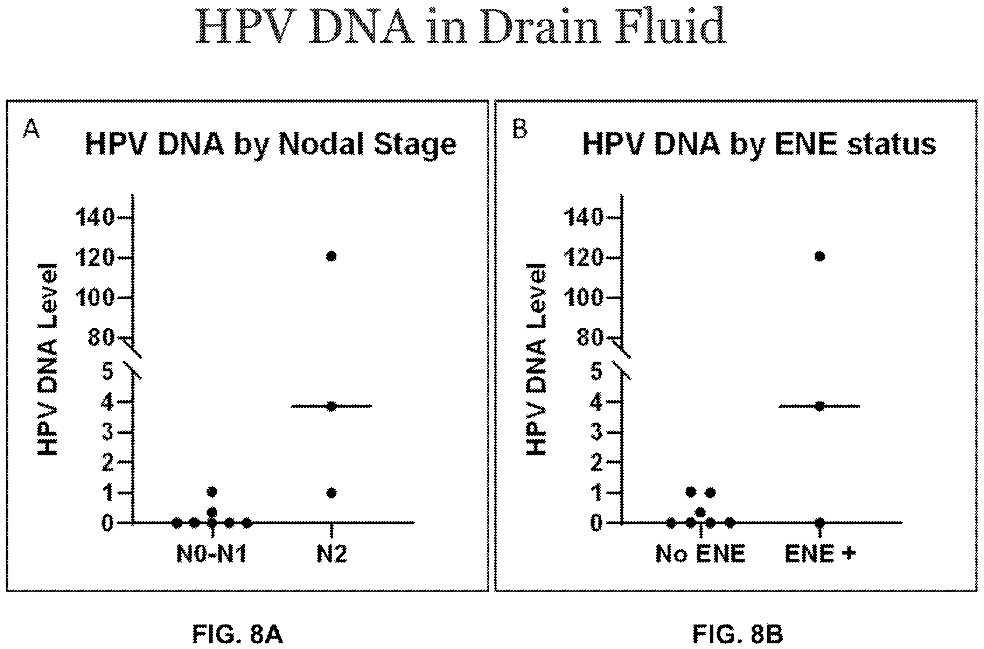

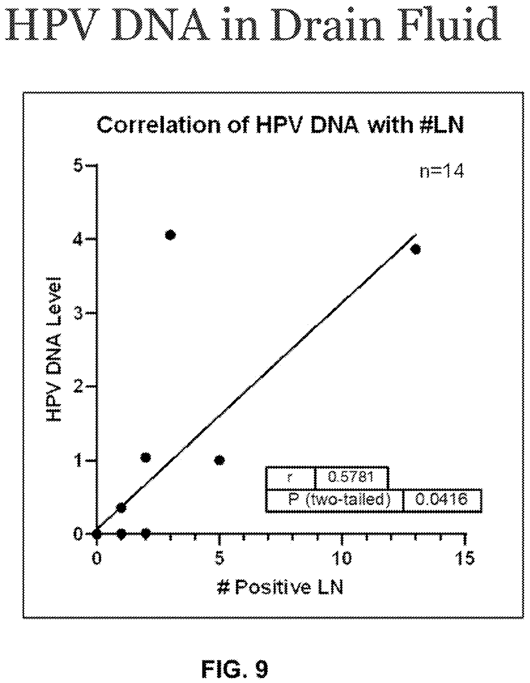

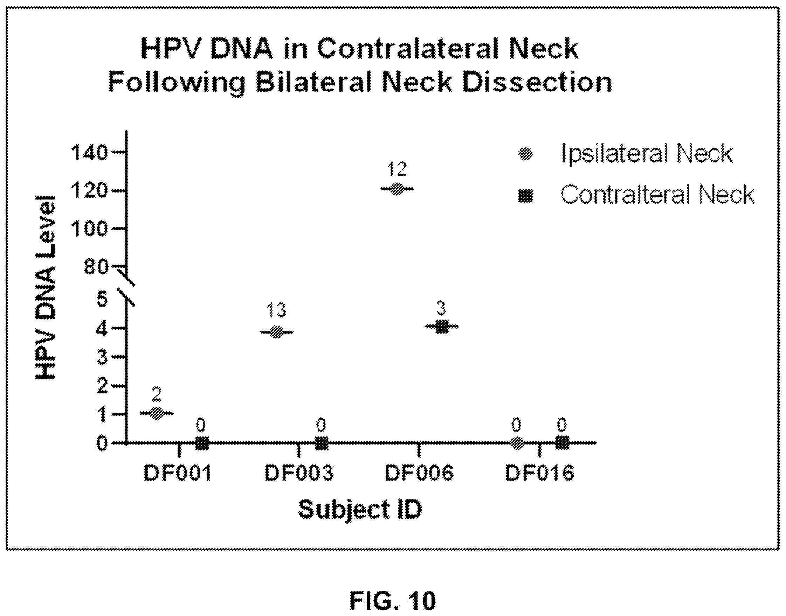

In various aspects, HPV DNA is measured within samples from drain fluid collected following neck dissection surgery. In other aspects, the presence or absence of residual HPV DNA after surgery are be used as a more proximal liquid biomarker than corresponding plasma-detected HPV DNA, and could help guide the need for postoperative radiation therapy or chemotherapy in the setting of treatment de-intensification. Preliminary data demonstrates that HPV DNA levels in drain fluid measured through real-time PCR correlated with the number of positive lymph nodes in the neck (see FIG. 8A and FIG. 9) as well as with extracapsular extension of cancer in the lymph nodes (see FIG. 8B). Additionally, patients undergoing neck dissection but who are found to have no pathologically-positive nodes show no detectable HPV in drain fluid. Further, patients undergoing bilateral neck dissection demonstrated different levels of HPV DNA within the samples obtained from each of the two post-surgical drain lines that were correlated with the nodal status of each side (see FIG. 10).

In various aspects, HPV DNA detected within drain samples may be used to guide radiation and chemotherapy dose after surgery as a potential means of further de-intensifying treatment. In these various aspects, detection of HPV DNA in the drain fluid may be used as a biomarker, wherein patients with detected HPV DNA in the drain fluid may be recommended for treatment de-intensifying. Using drain fluid as a liquid biopsy applies similar principles to those used to detect HPV cfDNA in plasma, but provides for a more proximal biomarker derived directly from the surgical bed. By way of non-limiting example, existing liquid biopsy tests such as NavDx^{™} used for the detection of circulating tumor DNA (ctDNA) may be modified for use in detected cfHPVDNA in drainage fluid samples.

In various aspects, the concept of using surgery drain fluid as a proximal marker to determine prognosis or adjuvant therapy for cancers could be expanded to other cancer sites. In addition to head and neck cancer, HPV causes cervical, penile, and anal cancer. In various aspects, HPV drainage fluids collected after surgical interventions of other HPV-associated cancers may be analyzed to detect cfHPVDNA to aid in assessing patient prognosis and selected an appropriate treatment plan. In another aspect, the methods described above may be used to detect other non-HPV biomarkers associated with other cancer types within drainage fluid collected after surgical interventions. By way of non-limiting example, thyroglobulin, a known biomarker for thyroid cancer, may be detected in drainage fluid after surgical neck dissection using a detection method such as ELISA and used to assess patient prognosis and/or select a treatment plan for thyroid cancer.

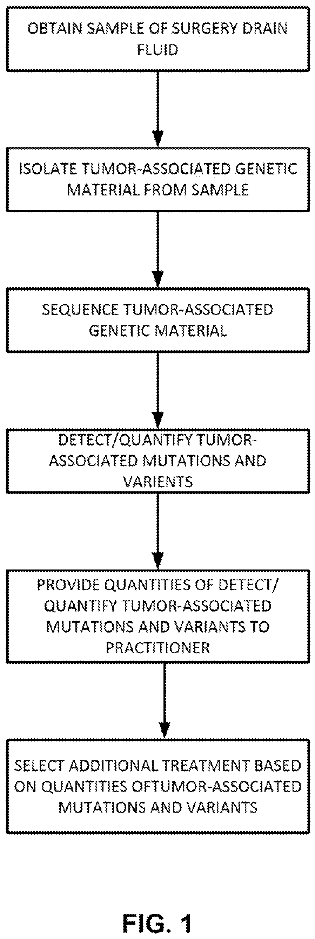

In various aspects, a method for detecting minimal residual disease in a subject following a cancer surgery is disclosed. FIG. 1 is a flow chart illustrating the steps of the disclosed method in one aspect. The method includes obtaining a surgical drainage sample from the subject. The surgical drainage sample is obtained using any known method including, but not limited to, capturing a surgical drainage tube associated with the cancer surgery. In some aspects, the sample may be obtained within about 24 hours of the completion of the surgery, providing the practitioner with timely information regarding tumor-related genetic material in the surgical drainage that may be used to select additional treatments.

In various aspects, the cancer surgery includes any surgery directed at removing cancerous tissues or tumors from the subject including, but not limited to, resectioning surgery, dissection surgery, excision surgery, and any combination thereof.

Referring again to FIG. 1, the method further includes isolating an amount of tumor-associated genetic material from the surgical drainage sample. In various aspects, the tumor-associated genetic material includes cell-free DNA, RNA, proteins, exosomes, and any combination thereof. In other aspects, the tumor-associated genetic material is produced or associated with a plurality of cancer cells. Non-limiting examples of cancer cells include oropharyngeal cancer cells, lung cancer cells, breast cancer cells, melanoma cells, colon cancer cells, thyroid cancer cells, prostate cancer cells, ovarian cancer cells, testicular cancer cells, penile cancer cells, cervical cancer cells, anal cancer cells, brain cancer cells, liver cancer cells, pancreatic cancer cells, and testicular cancer cells.

Additional non-limiting examples of cancer cells include cells from a variety of cancer types including Acute Lymphoblastic Leukemia (ALL); Acute Myeloid Leukemia (AML); Adrenocortical Carcinoma; AIDS-Related Cancers; Kaposi Sarcoma (Soft Tissue Sarcoma); AIDS-Related Lymphoma (Lymphoma); Primary CNS Lymphoma (Lymphoma); Anal Cancer; Appendix Cancer; Gastrointestinal Carcinoid Tumors; Astrocytomas; Atypical Teratoid/Rhabdoid Tumor, Childhood, Central Nervous System (Brain Cancer); Basal Cell Carcinoma of the Skin; Bile Duct Cancer; Bladder Cancer; Bone Cancer (including Ewing Sarcoma and Osteosarcoma and Malignant Fibrous Histiocytoma); Brain Tumors; Breast Cancer; Bronchial Tumors; Burkitt Lymphoma; Carcinoid Tumor (Gastrointestinal); Childhood Carcinoid Tumors; Cardiac (Heart) Tumors; Central Nervous System cancer; Atypical Teratoid/Rhabdoid Tumor, Childhood (Brain Cancer); Embryonal Tumors, Childhood (Brain Cancer); Germ Cell Tumor, Childhood (Brain Cancer); Primary CNS Lymphoma; Cervical Cancer; Cholangiocarcinoma; Bile Duct Cancer Chordoma; Chronic Lymphocytic Leukemia (CLL); Chronic Myelogenous Leukemia (CML); Chronic Myeloproliferative Neoplasms; Colorectal Cancer; Craniopharyngioma (Brain Cancer); Cutaneous T-Cell; Ductal Carcinoma In Situ (DCIS); Embryonal Tumors, Central Nervous System, Childhood (Brain Cancer); Endometrial Cancer (Uterine Cancer); Ependymoma, Childhood (Brain Cancer); Esophageal Cancer; Esthesioneuroblastoma; Ewing Sarcoma (Bone Cancer); Extracranial Germ Cell Tumor; Extragonadal Germ Cell Tumor; Eye Cancer; Intraocular Melanoma; Intraocular Melanoma; Retinoblastoma; Fallopian Tube Cancer; Fibrous Histiocytoma of Bone, Malignant, or Osteosarcoma; Gallbladder Cancer; Gastric (Stomach) Cancer; Gastrointestinal Carcinoid Tumor; Gastrointestinal Stromal Tumors (GIST) (Soft Tissue Sarcoma); Germ Cell Tumors; Central Nervous System Germ Cell Tumors (Brain Cancer); Childhood Extracranial Germ Cell Tumors; Extragonadal Germ Cell Tumors; Ovarian Germ Cell Tumors; Testicular Cancer; Gestational Trophoblastic Disease; Hairy Cell Leukemia; Head and Neck Cancer; Heart Tumors; Hepatocellular (Liver) Cancer; Histiocytosis, Langerhans Cell; Hodgkin Lymphoma; Hypopharyngeal Cancer; Intraocular Melanoma; Islet Cell Tumors; Pancreatic Neuroendocrine Tumors; Kaposi Sarcoma (Soft Tissue Sarcoma); Kidney (Renal Cell) Cancer; Langerhans Cell Histiocytosis; Laryngeal Cancer; Leukemia; Lip and Oral Cavity Cancer; Liver Cancer; Lung Cancer (Non-Small Cell and Small Cell); Lymphoma; Male Breast Cancer; Malignant Fibrous Histiocytoma of Bone or Osteosarcoma; Melanoma; Melanoma, Intraocular (Eye); Merkel Cell Carcinoma (Skin Cancer); Mesothelioma, Malignant; Metastatic Cancer; Metastatic Squamous Neck Cancer with Occult Primary; Midline Tract Carcinoma Involving NUT Gene; Mouth Cancer; Multiple Endocrine Neoplasia Syndromes; Multiple Myeloma/Plasma Cell Neoplasms; Mycosis Fungoides (Lymphoma); Myelodysplastic Syndromes, Myelodysplastic/Myeloproliferative Neoplasms; Myelogenous Leukemia, Chronic (CML); Myeloid Leukemia, Acute (AML); Myeloproliferative Neoplasms; Nasal Cavity and Paranasal Sinus Cancer; Nasopharyngeal Cancer; Neuroblastoma; Non-Hodgkin Lymphoma; Non-Small Cell Lung Cancer; Oral Cancer, Lip or Oral Cavity Cancer; Oropharyngeal Cancer; Osteosarcoma and Malignant Fibrous Histiocytoma of Bone; Ovarian Cancer Pancreatic Cancer; Pancreatic Neuroendocrine Tumors (Islet Cell Tumors); Papillomatosis; Paraganglioma; Paranasal Sinus and Nasal Cavity Cancer; Parathyroid Cancer; Penile Cancer; Pharyngeal Cancer; Pheochromocytoma; Pituitary Tumor; Plasma Cell Neoplasm/Multiple Myeloma; Pleuropulmonary Blastoma; Pregnancy and Breast Cancer; Primary Central Nervous System (CNS) Lymphoma; Primary Peritoneal Cancer; Prostate Cancer; Rectal Cancer; Recurrent Cancer Renal Cell (Kidney) Cancer; Retinoblastoma; Rhabdomyosarcoma, Childhood (Soft Tissue Sarcoma); Salivary Gland Cancer; Sarcoma; Childhood Rhabdomyosarcoma (Soft Tissue Sarcoma); Childhood Vascular Tumors (Soft Tissue Sarcoma); Ewing Sarcoma (Bone Cancer); Kaposi Sarcoma (Soft Tissue Sarcoma); Osteosarcoma (Bone Cancer); Uterine Sarcoma; Sézary Syndrome (Lymphoma); Skin Cancer; Small Cell Lung Cancer; Small Intestine Cancer; Soft Tissue Sarcoma; Squamous Cell Carcinoma of the Skin; Squamous Neck Cancer with Occult Primary, Metastatic; Stomach (Gastric) Cancer; T-Cell Lymphoma, Cutaneous; Lymphoma; Mycosis Fungoides and Sèzary Syndrome; Testicular Cancer; Throat Cancer; Nasopharyngeal Cancer; Oropharyngeal Cancer; Hypopharyngeal Cancer; Thymoma and Thymic Carcinoma; Thyroid Cancer; Thyroid Tumors; Transitional Cell Cancer of the Renal Pelvis and Ureter (Kidney (Renal Cell) Cancer); Ureter and Renal Pelvis; Transitional Cell Cancer (Kidney (Renal Cell) Cancer; Urethral Cancer; Uterine Cancer, Endometrial; Uterine Sarcoma; Vaginal Cancer; Vascular Tumors (Soft Tissue Sarcoma); Vulvar Cancer; or Wilms Tumor.

The tumor-associated genetic material may be isolated from the sample using any suitable method without limitation. Non-limiting examples of suitable isolation methods include filtering the sample, centrifuging the sample, contacting the sample with a chromatography medium, and any combination thereof.

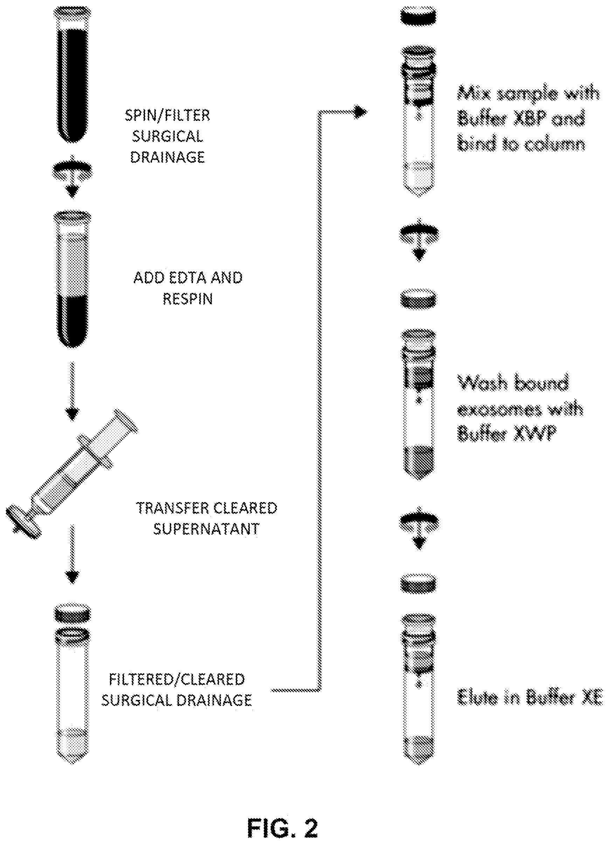

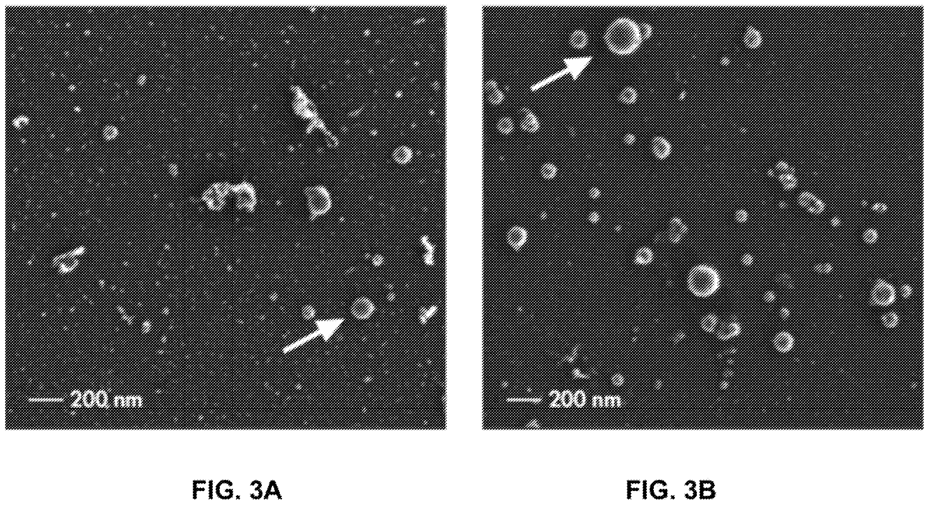

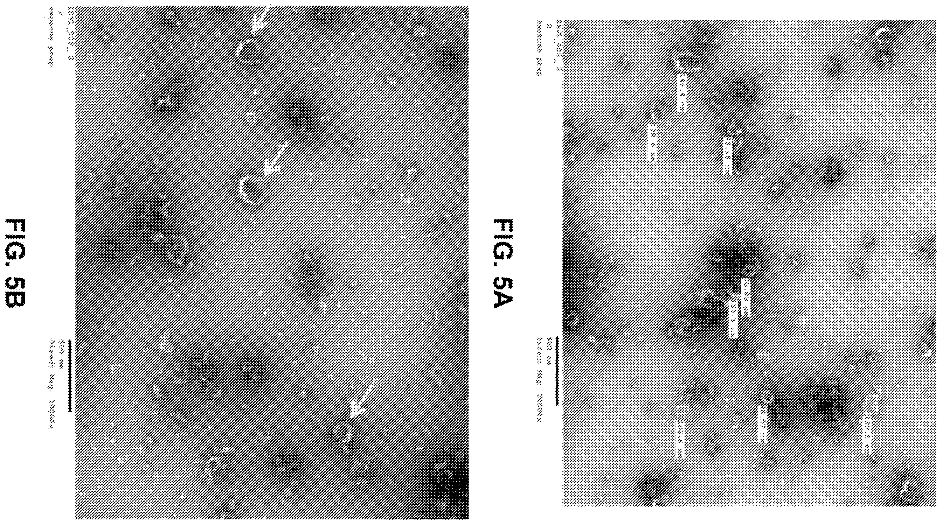

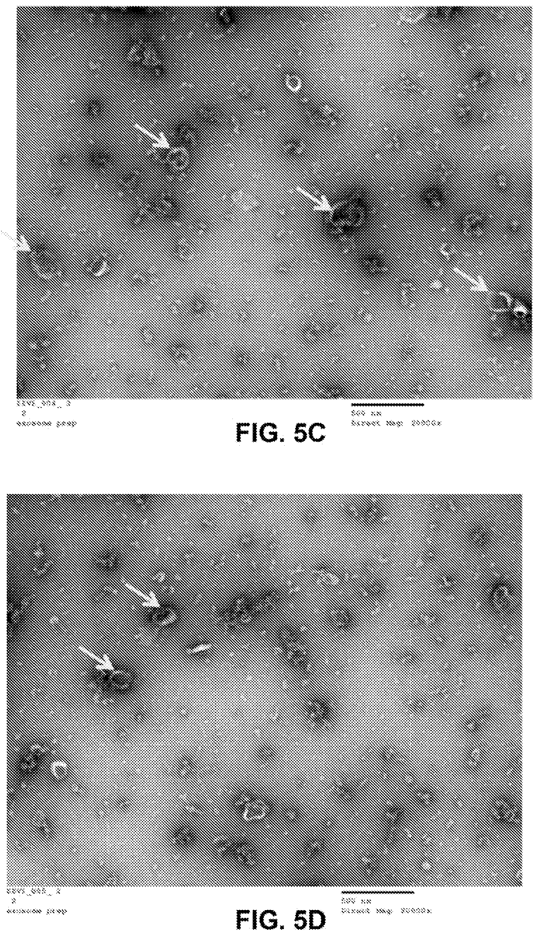

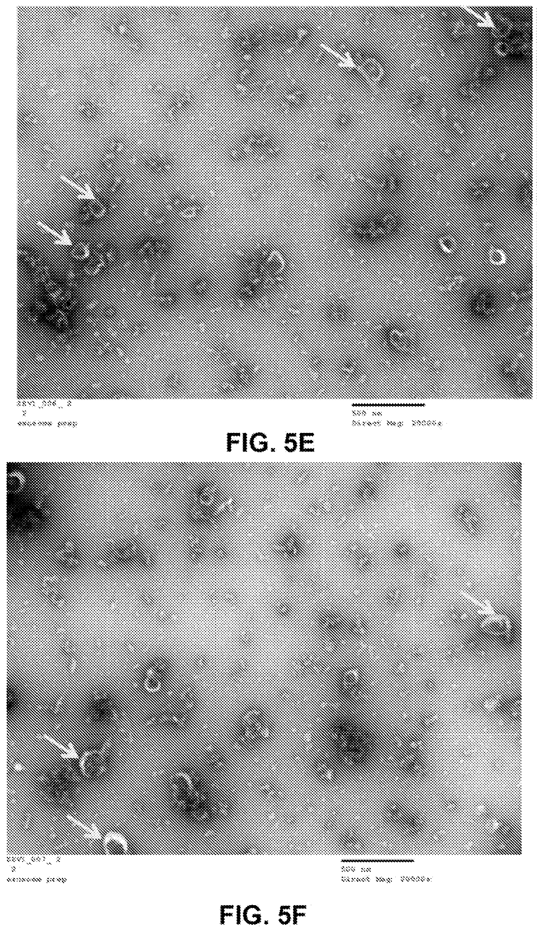

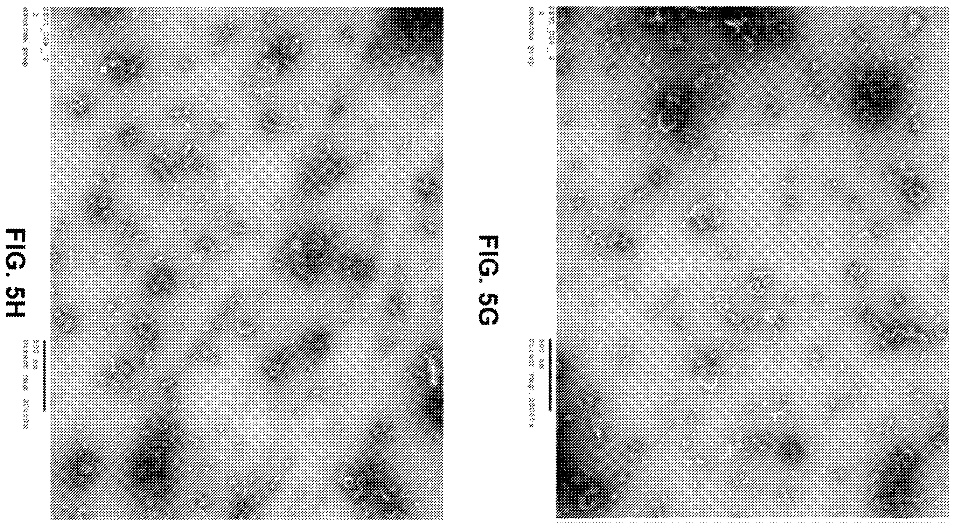

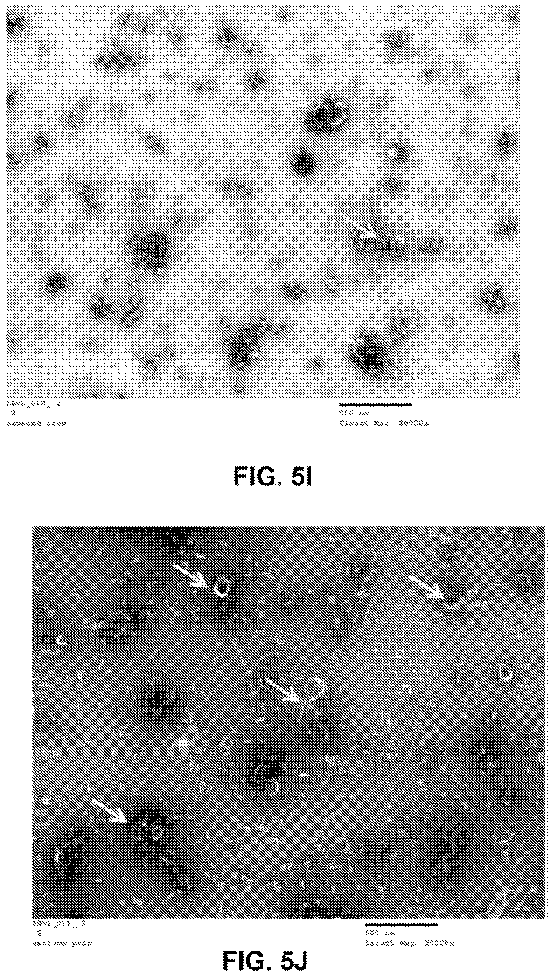

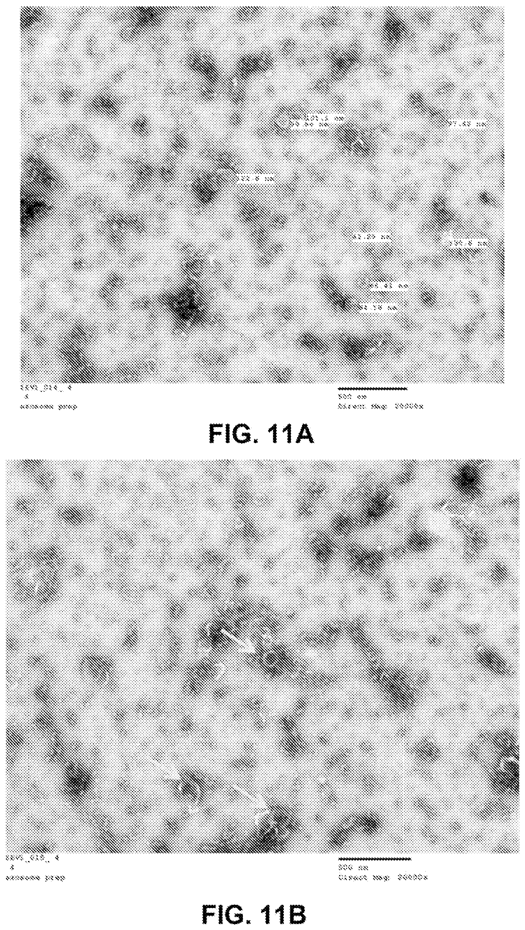

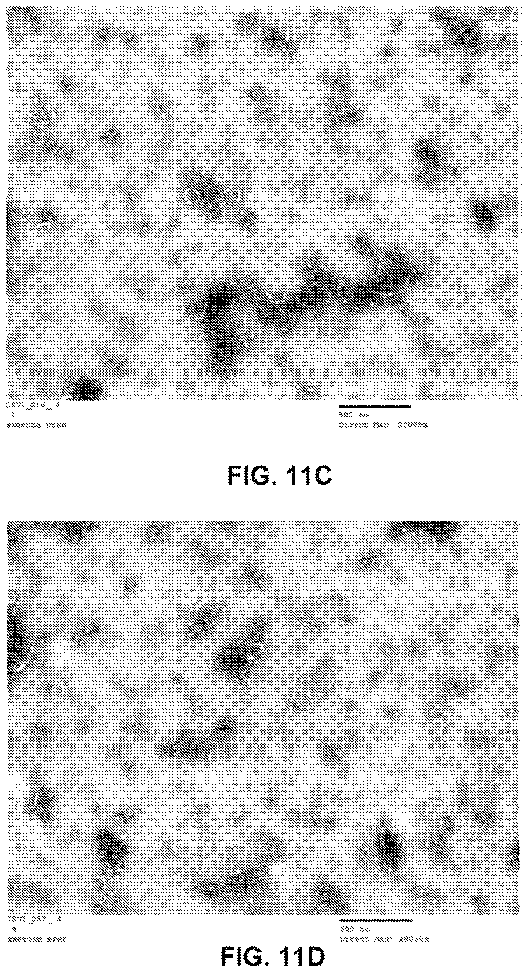

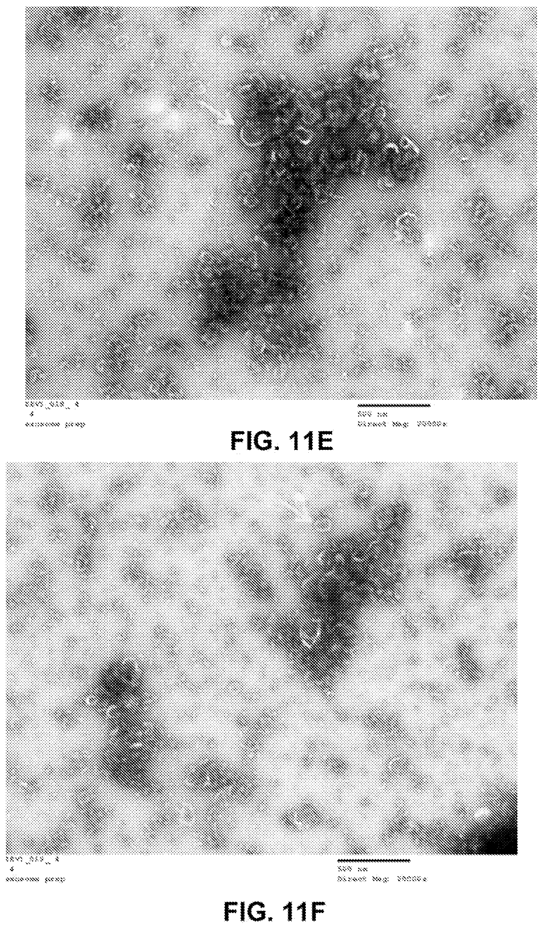

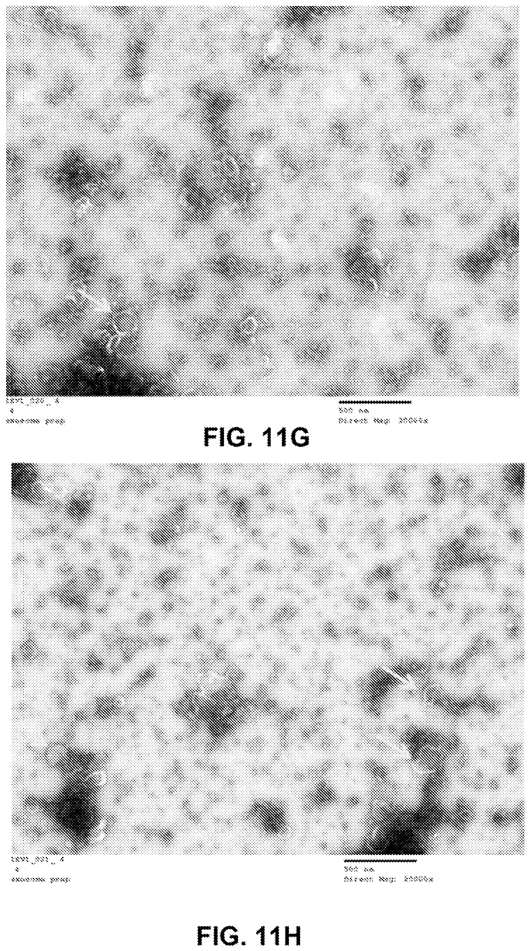

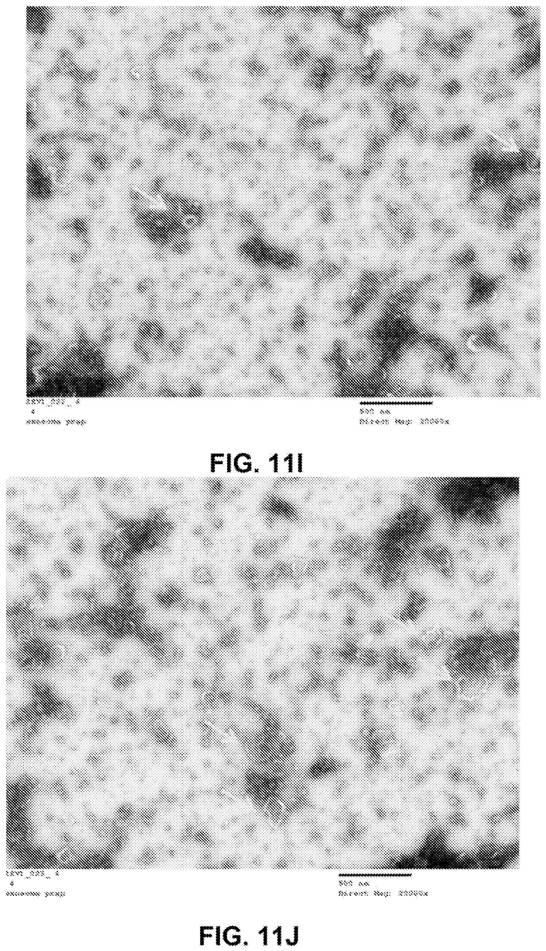

FIG. 2 is a schematic illustration of a method of isolating the tumor-associated genetic material from the sample in one aspect. As illustrated in FIG.2, the surgical drainage is centrifuged and filtered. EDTA is added to the sample to inhibit nucleases in the sample. The sample with added EDTA is further centrifuged, and the supernatant is removed and retained. In some aspects, the supernatant may be used as is for detection and quantification of cell-free DNA, RNA, and proteins. In other aspects, illustrated in FIG. 2, exosomes may be isolated from the supernatant by contacting the supernatant with a chromatographic media followed by elution. In various aspects, the isolation of the tumor-associated genetic material using the method illustrated in FIG. 2 yields intact exosomes, as illustrated in FIG. 3A, FIGS. 5A-5J, and 11A-11J. By ccontrast, other isolation methods such as ultracentrification may damage the exosomes, as illustrated in FIG. 3A.

Referring again to FIG. 1, the method further includes sequencing the tumor-associated genetic material in the sample and detecting/quantifying tumor-associated mutations and/or variants. Any suitable method may be used to sequence, detect, and quantify the tumor-associated mutations and/or variants including, but not limited to, next generation DNA sequencing, next generation RNA sequencing, next generation protein sequencing, PCR, Western blot, and any combination thereof. In some aspect, a targeted sequence assay panel may be used.

Referring again to FIG. 1, the method may further include providing the quantities of detected tumor-associated mutations and/or variants to a practitioner. Based on these detected quantities, the practitioner may make a determination of minimal residual disease in the patient, and/or select a follow-up treatment. Non-limiting examples of suitable follow-up treatments include radiotherapy, chemotherapy, follow-up surgery, active surveillance with imaging, and any combination thereof.

Definitions and methods described herein are provided to better define the present disclosure and to guide those of ordinary skill in the art in the practice of the present disclosure. Unless otherwise noted, terms are to be understood according to conventional usage by those of ordinary skill in the relevant art.

In some embodiments, numbers expressing quantities of ingredients, properties such as molecular weight, reaction conditions, and so forth, used to describe and claim certain embodiments of the present disclosure are to be understood as being modified in some instances by the term "about." In some embodiments, the term "about" is used to indicate that a value includes the standard deviation of the mean for the device or method being employed to determine the value. In some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the present disclosure are approximations, the numerical values set forth in the specific examples are reported as precisely as practicable. The numerical values presented in some embodiments of the present disclosure may contain certain errors necessarily resulting from the standard deviation found in their respective testing measurements. The recitation of ranges of values herein is merely intended to serve as a shorthand method of referring individually to each separate value falling within the range. Unless otherwise indicated herein, each individual value is incorporated into the specification as if it were individually recited herein. The recitation of discrete values is understood to include ranges between each value.

In some embodiments, the terms "a" and "an" and "the" and similar references used in the context of describing a particular embodiment (especially in the context of certain of the following claims) can be construed to cover both the singular and the plural, unless specifically noted otherwise. In some embodiments, the term "or" as used herein, including the claims, is used to mean "and/or" unless explicitly indicated to refer to alternatives only or the alternatives are mutually exclusive.

The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and can also cover other unlisted steps. Similarly, any composition or device that "comprises," "has" or "includes" one or more features is not limited to possessing only those one or more features and can cover other unlisted features.

All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the present disclosure and does not pose a limitation on the scope of the present disclosure otherwise claimed. No language in the specification should be construed as indicating any non-claimed element essential to the practice of the present disclosure.

Having described the present disclosure in detail, it will be apparent that modifications, variations, and equivalent embodiments are possible without departing the scope of the present disclosure defined in the appended claims. Furthermore, it should be appreciated that all examples in the present disclosure are provided as non-limiting examples.

## Claims

1. A method for detecting minimal residual disease in a subject following a cancer surgery, the method comprising:
a. isolating an amount of tumor-associated genetic material from a sample comprising a surgical drainage obtained from the subject ; wherein the amount of tumor-associated genetic material comprises cell-free DNA, RNA, proteins, exosomes, and any combination thereof;
b. sequencing the amount of tumor-associated genetic material to detect and quantify at least one tumor-associated mutation or variant in the amount of tumor-associated genetic material; and
c. providing the at least one quantity of the at least one tumor-associated mutation or variant to a practitioner, wherein the at least one tumor-associated mutation or variant is indicative of minimal residual disease in the subject.

2. The method of claim 1, wherein isolating the amount of tumor-associated genetic material from the sample further comprises filtering the sample, centrifuging the sample, contacting the sample with a chromatography medium, and any combination thereof.

3. The method of claim 1 or claim 2, further comprising selecting an additional treatment based on the quantity of the at least one tumor-associated mutation or variant,preferably wherein the additional treatment is selected from radiotherapy, chemotherapy, follow-up surgery, active surveillance with imaging, and any combination thereof.

4. The method of any preceding claim, wherein the tumor-associated genetic material is produced by a plurality of cancer cells, preferably wherein the plurality of cancer cells are selected from one of oropharyngeal cancer cells, lung cancer cells, breast cancer cells, melanoma cells, colon cancer cells, thyroid cancer cells, prostate cancer cells, ovarian cancer cells, testicular cancer cells, penile cancer cells, cervical cancer cells, anal cancer cells, brain cancer cells, liver cancer cells, pancreatic cancer cells, and testicular cancer cells.

5. The method of any preceding claim, wherein the cancer surgery is selected from a resectioning surgery, a dissection surgery, an excision surgery, and any combination thereof.

6. The method of any preceding claim, wherein the sample has been obtained within about 24 hours of the cancer surgery.

7. The method of any preceding claim, wherein the tumor-associated genetic material comprises cell-free HPV DNA (cfDNA) associated with oropharyngeal cancer cells preferably wherein the cell-free HPV DNA (cfDNA) comprises HPV16 DNA, HPV18 DNA, HPV31 DNA, HPV33 fragment, HPV35 fragment, HPV45 DNA, HPV52 DNA, HPV58 DNA, and any combination thereof.

8. The method of any preceding claim, wherein sequencing the amount of tumor-associated genetic material to detect and quantify at least one tumor-associated mutation or variant further comprises subjecting the sample to a sequencing method selected from next generation DNA sequencing, next generation RNA sequencing, next generation protein sequencing, PCR, Western blot, and any combination thereof.

9. A method for selecting a post-operative treatment for a cancer patient in need, the method comprising:
a. isolating an amount of tumor-associated genetic material from a sample comprising a surgical drainage ,wherein the amount of tumor-associated genetic material comprises cell-free DNA, RNA, proteins, exosomes, and any combination thereof;
b. sequencing the amount of tumor-associated genetic material to detect and quantify at least one tumor-associated mutation or variant in the amount of tumor-associated genetic material;
c. providing the at least one quantity of the at least one tumor-associated mutation or variant to a practitioner, wherein the at least one tumor-associated mutation or variant is indicative of minimal residual disease in the subject; and
d. selecting an additional treatment based on the quantity of the at least one tumor-associated mutation or variant, preferably wherein the additional treatment is selected from radiotherapy, chemotherapy, follow-up surgery, active surveillance with imaging, and any combination thereof.

10. The method of claim 9, wherein isolating the amount of tumor-associated genetic material from the sample further comprises filtering the sample, centrifuging the sample, contacting the sample with a chromatography medium, and any combination thereof.

11. The method of claim 9 or claim 10, wherein the tumor-associated genetic material is produced by a plurality of cancer cells, preferably wherein the plurality of cancer cells are selected from one of oropharyngeal cancer cells, lung cancer cells, breast cancer cells, melanoma cells, colon cancer cells, thyroid cancer cells, prostate cancer cells, ovarian cancer cells, testicular cancer cells, penile cancer cells, cervical cancer cells, anal cancer cells, brain cancer cells, liver cancer cells, pancreatic cancer cells, and testicular cancer cells.

12. The method of any one of claims 9-11, wherein the tumor-associated genetic material comprises cell-free HPV DNA (cfDNA) associated with oropharyngeal cancer cells.

13. The method of any one of claims 9-12, wherein sequencing the amount of tumor-associated genetic material to detect and quantify at least one tumor-associated mutation or variant further comprises subjecting the sample to a sequencing method selected from next generation DNA sequencing, next generation RNA sequencing, next generation protein sequencing , PCR, Western blot, and any combination thereof.

14. The method of any one of claims 9-13, wherein sequencing the amount of tumor-associated genetic material to detect and quantify at least one tumor-associated mutation or variant in the amount of tumor-associated genetic material further comprises detecting and quantifying at least one HPV strain associated with HPV(+) oropharyngeal cancer comprising HPV16 DNA, HPV18 DNA, HPV31 DNA, HPV33 fragment, HPV35 fragment, HPV45 DNA, HPV52 DNA, HPV58 DNA, and any combination thereof.

## Patentansprüche

1. Verfahren zum Nachweisen minimaler Restkrankheit bei einem Patienten nach einer Krebsoperation, das Verfahren umfassend:
a. Isolieren einer Menge tumorassoziierten genetischen Materials aus einer Probe, die eine chirurgische Drainage umfasst, die von dem Patienten gewonnen wurde; wobei die Menge an tumorassoziiertem genetischem Material zellfreie DNA, RNA, Proteine, Exosomen und jede Kombination davon umfasst;
b. Sequenzieren der Menge tumorassoziierten genetischen Materials, um mindestens eine tumorassoziierte Mutation oder Variante in der Menge tumorassoziierten genetischen Materials nachzuweisen und zu quantifizieren; und
c. Bereitstellen der mindestens einen Menge der mindestens einen tumorassoziierten Mutation oder Variante an einen Arzt, wobei die mindestens eine tumorassoziierte Mutation oder Variante auf eine minimale Restkrankheit in dem Patienten hinweist.

2. Verfahren nach Anspruch 1, wobei das Isolieren der Menge an tumorassoziiertem genetischem Material aus der Probe ferner das Filtern der Probe, das Zentrifugieren der Probe, das Inkontaktbringen der Probe mit einem Chromatographiemedium und jede Kombination davon umfasst.

3. Verfahren nach Anspruch 1 oder Anspruch 2, ferner umfassend das Auswählen einer zusätzlichen Behandlung auf der Grundlage der Menge der mindestens einen tumorassoziierten Mutation oder Variante, wobei die zusätzliche Behandlung vorzugsweise aus Strahlentherapie, Chemotherapie, Folgeoperation, aktiver Überwachung mit Bildgebung und einer beliebigen Kombination davon ausgewählt wird.

4. Verfahren nach einem der vorhergehenden Ansprüche, wobei das tumorassoziierte genetische Material von einer Vielzahl von Krebszellen produziert wird, wobei die Vielzahl von Krebszellen vorzugsweise ausgewählt ist aus Oropharynxkarzinomzellen, Lungenkarzinomzellen, Brustkrebszellen, Melanomzellen, Kolonkarzinomzellen, Schilddrüsenkarzinomzellen, Prostatakarzinomzellen, Ovarialkarzinomzellen, Hodenkarzinomzellen, Peniskarzinomzellen, Zervixkarzinomzellen, Analkarzinomzellen, Hirntumorzellen, Leberkarzinomzellen, Pankreaskarzinomzellen und Hodenkarzinomzellen.

5. Verfahren nach einem der vorhergehenden Ansprüche, wobei die Krebsoperation ausgewählt ist aus einer Resektionsoperation, einer Dissektionsoperation, einer Exzisionsoperation und einer beliebigen Kombination davon.

6. Verfahren nach einem der vorhergehenden Ansprüche, wobei die Probe innerhalb von etwa 24 Stunden nach der Krebsoperation gewonnen wurde.

7. Verfahren nach einem der vorhergehenden Ansprüche, wobei das tumorassoziierte genetische Material zellfreies HPV-DNA (cfDNA) umfasst, das mit oropharyngealen Krebszellen assoziiert ist, wobei das zellfreie HPV-DNA (cfDNA) vorzugsweise HPV16-DNA, HPV VI8-DNA, HPV31-DNA, HPV33-Fragment, HPV35-Fragment, HPV45-DNA, HPV52-DNA, HPV58-DNA und eine beliebige Kombination davon umfasst.

8. Verfahren nach einem der vorhergehenden Ansprüche, wobei das Sequenzieren der Menge an tumorassoziiertem genetischem Material zum Nachweisen und Quantifizieren mindestens einer tumorassoziierten Mutation oder Variante ferner umfasst, dass die Probe einem Sequenzierungsverfahren unterzogen wird, das ausgewählt ist aus DNA-Sequenzierung der nächsten Generation, RNA-Sequenzierung der nächsten Generation, Proteinsequenzierung der nächsten Generation, PCR, Western Blot und einer beliebigen Kombination davon.

9. Verfahren zum Auswählen einer postoperativen Behandlung für einen Krebspatienten, der dieser bedarf, das Verfahren umfassend:
a. Isolieren einer Menge an tumorassoziiertem genetischem Material aus einer Probe, die eine chirurgische Drainage umfasst, wobei die Menge an tumorassoziiertem genetischem Material zellfreie DNA, RNA, Proteine, Exosomen und beliebige Kombinationen davon umfasst;
b. Sequenzieren der Menge tumorassoziierten genetischen Materials, um mindestens eine tumorassoziierte Mutation oder Variante in der Menge tumorassoziierten genetischen Materials nachzuweisen und zu quantifizieren;
c. Bereitstellen der mindestens einen Menge der mindestens einen tumorassoziierten Mutation oder Variante an einen Arzt, wobei die mindestens eine tumorassoziierte Mutation oder Variante auf eine minimale Restkrankheit in dem Patienten hinweist; und
d. Auswählen einer zusätzlichen Behandlung auf der Grundlage der Menge der mindestens einen tumorassoziierten Mutation oder Variante, wobei die zusätzliche Behandlung vorzugsweise aus Strahlentherapie, Chemotherapie, Folgeoperation, aktiver Überwachung mit Bildgebung und einer beliebigen Kombination davon ausgewählt wird.

10. Verfahren nach Anspruch 9, wobei das Isolieren der Menge an tumorassoziiertem genetischem Material aus der Probe ferner das Filtern der Probe, das Zentrifugieren der Probe, das Inkontaktbringen der Probe mit einem Chromatographiemedium und jede Kombination davon umfasst.

11. Verfahren nach Anspruch 9 oder Anspruch 10, wobei das tumorassoziierte genetische Material von einer Vielzahl von Krebszellen produziert wird, wobei die Vielzahl von Krebszellen vorzugsweise ausgewählt ist aus Oropharynxkarzinomzellen, Lungenkarzinomzellen, Brustkrebszellen, Melanomzellen, Kolonkarzinomzellen, Schilddrüsenkarzinomzellen, Prostatakarzinomzellen, Ovarialkarzinomzellen, Hodenkarzinomzellen, Peniskarzinomzellen, Zervixkarzinomzellen, Analkarzinomzellen, Hirntumorzellen, Leberkarzinomzellen, Pankreaskarzinomzellen und Hodenkarzinomzellen.

12. Verfahren nach einem der Ansprüche 9 bis 11, wobei das tumorassoziierte genetische Material zellfreie HPV-DNA (cfDNA) umfasst, die mit oropharyngealen Krebszellen assoziiert ist.

13. Verfahren nach einem der Ansprüche 9-12, wobei das Sequenzieren der Menge an tumorassoziiertem genetischem Material zum Nachweisen und Quantifizieren mindestens einer tumorassoziierten Mutation oder Variante ferner umfasst, dass die Probe einem Sequenzierungsverfahren unterzogen wird, das ausgewählt ist aus DNA-Sequenzierung der nächsten Generation, RNA-Sequenzierung der nächsten Generation, Proteinsequenzierung der nächsten Generation, PCR, Western Blot und einer beliebigen Kombination davon.

14. Verfahren nach einem der Ansprüche 9 bis 13, wobei das Sequenzieren der Menge an tumorassoziiertem genetischem Material zum Nachweisen und Quantifizieren mindestens einer tumorassoziierten Mutation oder Variante in der Menge an tumorassoziiertem genetischem Material ferner das Nachweisen und Quantifizieren mindestens eines HPV-Stamms umfasst, der mit HPV(+)-Oropharynxkarzinom assoziiert ist, umfassend HPV16-DNA, HPV18-DNA, HPV31-DNA, HPV33-Fragment, HPV35-Fragment, HPV45-DNA, HPV52-DNA, HPV58-DNA und beliebige Kombinationen davon.

## Revendications

1. Procédé pour détecter une maladie résiduelle minimale chez un sujet après une chirurgie cancéreuse, le procédé comprenant les étapes consistant à :
a. isoler une quantité de matériel génétique associé à une tumeur à partir d'un échantillon comprenant un drainage chirurgical obtenu auprès du sujet ; la quantité de matériel génétique associé à une tumeur comprenant de l'ADN acellulaire, de l'ARN, des protéines, des exosomes, et toute association de ceux-ci ;
b. séquencer la quantité de matériel génétique associé à une tumeur pour détecter et quantifier au moins une mutation ou un variant associé(e) à une tumeur dans la quantité de matériel génétique associé à une tumeur ; et
c) fournir à un praticien ladite au moins une quantité de ladite/dudit au moins une mutation ou un variant associé(e) à une tumeur, ladite/ledit au moins une mutation ou un variant associé(e) à une tumeur étant indicatif(ve) de maladie résiduelle minimale chez le sujet.

2. Procédé selon la revendication 1, dans lequel isoler la quantité de matériel génétique associé à une tumeur à partir de l'échantillon comprend en outre filtrer l'échantillon, centrifuger l'échantillon, mettre l'échantillon en contact avec un milieu chromatographique, et toute combinaison de ceux-ci.

3. Procédé selon la revendication 1 ou la revendication 2, comprenant en outre sélectionner un traitement supplémentaire sur la base de la quantité de ladite/dudit au moins une mutation ou un variant associé(e) à une tumeur, de préférence le traitement supplémentaire étant choisi parmi la radiothérapie, la chimiothérapie, la chirurgie de suivi, la surveillance active par imagerie, et toute combinaison de celles-ci.

4. Procédé selon l'une quelconque des revendications précédentes, dans lequel le matériel génétique associé à une tumeur est produit par une pluralité de cellules cancéreuses, de préférence dans lequel la pluralité de cellules cancéreuses sont choisies parmi des cellules de cancer oropharyngé, des cellules de cancer pulmonaire, des cellules de cancer mammaire, des cellules de mélanome, des cellules de cancer du côlon, des cellules de cancer de la thyroïde, des cellules de cancer de la prostate, des cellules de cancer de l'ovaire, des cellules de cancer du testicule, des cellules de cancer pénien, des cellules de cancer du col utérin, des cellules de cancer anal, des cellules de cancer du cerveau, des cellules de cancer du foie, des cellules de cancer du pancréas, et des cellules de cancer du testicule.

5. Procédé selon l'une quelconque des revendications précédentes, dans lequel la chirurgie du cancer est choisie parmi une chirurgie de résection, une chirurgie de dissection, une chirurgie d'excision, et toute combinaison de celles-ci.

6. Procédé selon l'une quelconque des revendications précédentes, dans lequel l'échantillon a été obtenu dans un délai d'environ 24 heures après la chirurgie du cancer.

7. Procédé selon l'une quelconque des revendications précédentes, dans lequel le matériel génétique associé à une tumeur comprend de l'ADN de HPV acellulaire (cfDNA) associé à des cellules de cancer oropharyngé, de préférence dans lequel l'ADN de HPV acellulaire (cfDNA) comprend de l'ADN de HPV16, de l'ADN de HPV18, de l'ADN de HPV31, un fragment de HPV33, un fragment de HPV35, de l'ADN de HPV45, de l'ADN de HPV52, de l'ADN de HPV58, et toute association de ceux-ci.

8. Procédé selon l'une quelconque des revendications précédentes, dans lequel séquencer la quantité de matériel génétique associé à une tumeur pour détecter et quantifier au moins une mutation ou un variant associé(e) à une tumeur comprend en outre soumettre l'échantillon à une méthode de séquençage choisie parmi le séquençage d'ADN nouvelle génération, le séquençage d'ARN nouvelle génération, le séquençage de protéines nouvelle génération, la PCR, le Western blot, et toute combinaison de telles méthodes.

9. Procédé pour sélectionner un traitement postopératoire pour un patient cancéreux nécessitant un tel traitement, le procédé comprenant les étapes consistant à :
a. isoler une quantité de matériel génétique associé à une tumeur à partir d'un échantillon comprenant un drainage chirurgical, la quantité de matériel génétique associé à une tumeur comprenant de l'ADN acellulaire, de l'ARN, des protéines, des exosomes, et toute association de ceux-ci ;
b. séquencer la quantité de matériel génétique associé à une tumeur pour détecter et quantifier au moins une mutation ou un variant associé(e) à une tumeur dans la quantité de matériel génétique associé à une tumeur ;
c) fournir à un praticien ladite au moins une quantité de ladite/dudit au moins une mutation ou un variant associé(e) à une tumeur, ladite/ledit au moins une mutation ou un variant associé(e) à une tumeur étant indicatif(ve) de maladie résiduelle minimale chez le sujet ; et
d) sélectionner un traitement supplémentaire sur la base de la quantité de ladite/dudit au moins une mutation ou un variant associé(e) à une tumeur, de préférence le traitement supplémentaire étant choisi parmi la radiothérapie, la chimiothérapie, la chirurgie de suivi, la surveillance active par imagerie, et toute combinaison de celles-ci.

10. Procédé selon la revendication 9, dans lequel isoler la quantité de matériel génétique associé à une tumeur à partir de l'échantillon comprend en outre filtrer l'échantillon, centrifuger l'échantillon, mettre l'échantillon en contact avec un milieu chromatographique, et toute combinaison de ceux-ci.

11. Procédé selon la revendication 9 ou la revendication 10, dans lequel le matériel génétique associé à une tumeur est produit par une pluralité de cellules cancéreuses, de préférence dans lequel la pluralité de cellules cancéreuses sont choisies parmi des cellules de cancer oropharyngé, des cellules de cancer pulmonaire, des cellules de cancer mammaire, des cellules de mélanome, des cellules de cancer du côlon, des cellules de cancer de la thyroïde, des cellules de cancer de la prostate, des cellules de cancer de l'ovaire, des cellules de cancer du testicule, des cellules de cancer pénien, des cellules de cancer du col utérin, des cellules de cancer anal, des cellules de cancer du cerveau, des cellules de cancer du foie, des cellules de cancer du pancréas, et des cellules de cancer du testicule.

12. Procédé selon l'une quelconque des revendications 9-11, dans lequel le matériel génétique associé à une tumeur comprend de l'ADN de HPV acellulaire (cfDNA) associé à des cellules de cancer oropharyngé.

13. Procédé selon l'une quelconque des revendications 9-12, dans lequel séquencer la quantité de matériel génétique associé à une tumeur pour détecter et quantifier au moins une mutation ou un variant associé(e) à une tumeur comprend en outre soumettre l'échantillon à une méthode de séquençage choisie parmi le séquençage d'ADN nouvelle génération, le séquençage d'ARN nouvelle génération, le séquençage de protéines nouvelle génération, la PCR, le Western blot, et toute combinaison de telles méthodes.

14. Procédé selon l'une quelconque des revendications 9-13, dans lequel séquencer la quantité de matériel génétique associé à une tumeur pour détecter et quantifier au moins une mutation ou un variant associé(e) à une tumeur dans la quantité de matériel génétique associé à une tumeur comprend en outre détecter et quantifier au moins une souche de HPV associée au cancer oropharyngé HPV(+) comprenant de l'ADN de HPV16, de l'ADN de HPV18, de l'ADN de HPV31, un fragment de HPV33, un fragment de HPV35, de l'ADN de HPV45, de l'ADN de HPV52, de l'ADN de HPV58, et toute association de ceux-ci.
